(19) Europäisches Patentamt
European Patent Office
Office européen des brevets

(11) Veröffentlichungsnummer: **0 027 655**
**B1**

(12) # EUROPÄISCHE PATENTSCHRIFT

(45) Veröffentlichungstag der Patentschrift: **07.03.84**

(51) Int. Cl.³: **A 61 K 7/06**

(21) Anmeldenummer: **80106354.6**

(22) Anmeldetag: **18.10.80**

(54) **Verwendung von kosmetischen Mitteln zur Behandlung der Haare und der Kopfhaut.**

(30) Priorität: **23.10.79 DE 2942666**

(43) Veröffentlichungstag der Anmeldung: **29.04.81 Patentblatt 81/17**

(45) Bekanntmachung des Hinweises auf die Patenterteilung: **07.03.84 Patentblatt 84/10**

(84) Benannte Vertragsstaaten: **AT BE CH DE FR GB IT LI NL SE**

(56) Entgegenhaltungen:
**FR - A - 2 123 502**
**US - A - 2 986 573**
**US - A - 4 184 039**

**O.A. NEUMÜLLER: "Römpps Chemie-Lexikon", 7. Auflage, Band 6, 1977, Franckh'sche Verlagshandlung, W. Keller & Co., Seite 3566, Suttgart, DE**
**THE MERCK INDEX, 8. Auflage, 1968, Seite 343, Merck & Co. Rahway, N.J., U.S.A.**

(73) Patentinhaber: **Wella Aktiengesellschaft, Berliner Allee 65, D-6100 Darmstadt (DE)**

(72) Erfinder: **Noser, Friedrich, Dr., La Haita, CH-1711 Bonnefontaine (CH)**

**EP 0 027 655 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verwendung von kosmetischen Mitteln zur Behandlung der Haare und der Kopfhaut

Die Erfindung betrifft die Verwendung von kosmetischen Mitteln mit einem Gehalt an mindestens einem [Benzo-1,2,4-thiadiazin]-1-dioxid-derivat als haarwuchsfördernden Wirkstoff.

Es sind schon sehr zahlreiche und unterschiedliche Substanzen zur Förderung des Haarwachstums empfohlen worden. So hat man bereits verschiedene Verbindungen vorgeschlagen, die infolge ihrer keratolytischen Wirkung eine vermehrte Bildung von Schuppen und das oft damit verbundene Nachlassen des Haarwachstums verhindern sollen. Hierzu gehören insbesondere Salicylsäure, Resorcin, Benzoxathiol und Hexachlorophen, welche gleichzeitig auch eine bakterizide und antimykotische Wirkung aufweisen. Ferner wurden schon für eine Art Reiztherapie des Haarbodens verschiedene Stoffe verwendet, um die Durchblutung der Kopfhaut zu steigern und dadurch den Haarwuchs anzuregen. Von diesen Stoffen seien hauptsächlich Nicotinsäure, Derivate der Nicotinsäure, Cantharidin, Pyrogallol, Pilocarpin, Ameisensäure und Histamin erwähnt. Weiterhin sind auch schwefelhaltige Präparate, Hormone unterschiedlicher Herkunft, Vitamine des B-Komplexes wie insbesondere Pantothensäure, Lecithin- und Keratinpräparate, Trichosaccharide, Cholesterin und andere gegen Haarausfall empfohlen worden.

Die gegenwärtig für eine Verwendung zur Haarwuchsförderung bekannten Substanzen zeigen jedoch, insbesondere beim männlichen Haarausfall, bis auf sehr wenige Behandlungsfälle entweder keine oder nur eine sehr geringe Wirksamkeit.

Aus dem Merck Index, 8. Auflage (1968) Seite 343 ist bekannt, dass die Verbindung 7-Chlor-3-methyl-2H-1,2,4-benzothiadiazin in der Medizin als non-diuretisch-antihypertensiver Wirkstoff verwendet wird. Die Herstellung dieser Verbindung betreffende Literatur ist dort ebenfalls genannt. Ferner ist in der US-Patentschrift 2 986 573 die Herstellung bestimmter substituierter 1,2,4-Benzothiadiazin-1,1-dioxide, unter anderem von 7-Chlor-3-methyl-2H-1,2,4-benzothiadiazin, beschrieben. Gemäss der Lehre der US-Patentschrift sollen die dort genannten substituierten 1,2,4-Benzothiadiazin-1,1-dioxide als antihypertensive Wirkstoffe mit pharmazeutischen Trägerstoffen, oral oder parenteral angewendet, zur Therapie von Bluthochdruck eingesetzt werden.

Über die Möglichkeit, substituierte 1,2,4-Benzothiadiazin-1,1-dioxide äusserlich am Haar und der Kopfhaut des Menschen zur Behandlung von Haarausfall zu verwenden, wurde jedoch in der Literatur bisher nichts berichtet.

Demgegenüber wurde nun überraschend gefunden, dass die Verwendung eines kosmetischen Mittels auf der Grundlage üblicher kosmetischer Träger- und Zusatzstoffe, gekennzeichnet durch den Gehalt an mindestens einem [Benzo-1,2,4-thiadiazin]-1-dioxid-derivat der allgemeinen Formel

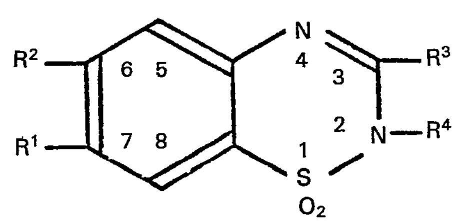

(I)

in der

$R^1$ die Bedeutung Cl, $CF_3$ oder $-SO_2NH_2$ hat,

$R^2$ einer der Reste H, Cl, $-SO_2NH_2$ ist, wobei jedoch die Reste $R^1$ und $R^2$ nicht beide gleichzeitig Cl oder $-SO_2NH_2$ bedeuten,

$R^3$ einen der Reste H, $C_1$- bis $C_3$-Alkyl, $CH_2Cl$, $CHCl_2$, $CCl_3$, COOH, $-CH_2OH$, $-CH_2OSO_3H$, $-CH_2-CH(CH_3)_2$, $-CH_2-S-CH_2-CF_3$, $-(CH_2)_4-CH_3$, $-CH_2-S-CH_2-CH=CH_2$, $-CH_2-S-CH_2-CH_3$, $-CH(CH_3)-CH(CH_3)-CH_2-CH_3$,

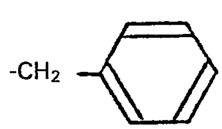 , 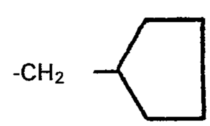 , 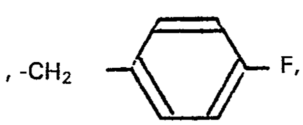,

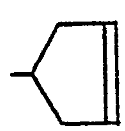 , 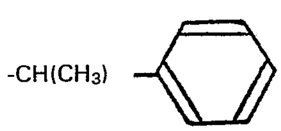 , 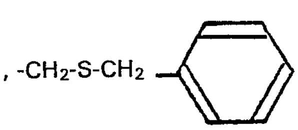

und 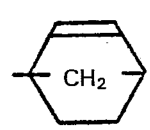 darstellt und

$R^4$ die Bedeutung H oder $CH_3$ hat, zur Behandlung der Kopfhaut und der Haare nicht nur der Haarausfall gestoppt wird, sondern auch das Wachstum des Kopfhaares deutlich gefördert wird.

Von den für eine erfindungsgemässe Verwendung in den kosmetischen Mitteln enthaltenen geeigneten [Benzo-1,2,4-thiadiazin]-1-dioxid-derivaten sind besonders bevorzugt solche der allgemeinen Formel

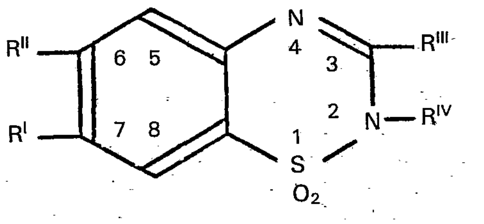

(II)

in der

$R^I$ die Bedeutung Cl, $CF_3$ oder $-SO_2NH_2$ hat,

$R^{II}$ einer der Reste H, Cl oder $-SO_2NH_2$ ist, wobei jedoch die Reste $R^I$ und $R^{II}$ nicht beide gleichzeitig Cl oder $-SO_2NH_2$ bedeuten,

$R^{III}$ einen der Reste $CH_3$, $CH_2OH$ oder COOH darstellt und

$R^{IV}$ die Bedeutung H oder $CH_3$ hat.

Beispiele für Verbindungen gemäss Formel (II) sind

7-Chlor-3-methyl-2H-[benzo-1,2,4-thiadiazin]-1--dioxid

7-Chlor-3-hydroxymethyl-2H-[benzo-1,2,4-thiadiazin]-1-dioxid und

7-Chlor-3-carboxy-2H-[benzo-1,2,4-thiadiazin]-1--dioxid.

In den erfindungsgemäss zu verwendenden Mitteln können die Verbindungen gemäss Formel (I), von denen gegebenenfalls auch Gemische einsetzbar sind, in gelöstem, emulgiertem oder dispergiertem Zustand in geeigneten Trägerstoffen vorliegen. Als Trägerstoffe kommen im allgemeinen solche in Betracht, welche die Wirkstoffkomponente nicht nachteilig beeinflussen und gleichzeitig unschädlich gegenüber der menschlichen Haut sind. Solche Trägerstoffe sind beispielsweise Wasser, Ethanol und Isopropanol, insbesondere aber wegen der geringen Löslichkeit der [Benzo-1,2,4-thiadiazin]-1-dioxidderivate in den vorstehend genannten Trägerstoffen, Gemische aus Dimethylsulfoxid mit diesen Trägerstoffen. Zur Herstellung von 1 bis 4%igen Lösungen der Verbindung 7-Chlor-3-methyl-2H-[benzo--1,2,4-thiadiazin]-1-dioxid eignen sich beispielsweise Dimethylsulfoxid/Ethanol-Gemische vom Verhältnis etwa 1 : 3, Dimethylsulfoxid/Isopropanol-Gemische vom Verhältnis etwa 1 : 9 und Dimethylsulfoxid/Wasser-Gemische vom Verhältnis etwa 1 : 1. Hierbei kann die Lösungseigenschaft der Gemische jeweils durch die Erhöhung des Dimethylsulfoxid-Anteils weiter gesteigert werden.

Die Zubereitungsform der kosmetischen Mittel kann beispielsweise die eines Einlegemittels, eines Rinses, eines Frisiergels, eines Haarsprays, eines Haaröls, einer Haarpomade, einer Haarkur, einer Wasserwellotion, eines Puders, insbesondere aber einer Haarcreme, eines Shampoos und eines Haarwassers, sein.

Die Konzentration der Verbindungen nach Formel (I) beträgt in den Zubereitungen, die auf dem Haar und der Kopfhaut verbleiben, wie zum Beispiel in Haarwässern und Einlegemitteln, 0,01 bis 5 Gew.-%, vorzugswseise 0,1 bis 4 Gew.-%. Zubereitungen, die kurz nach ihrer Anwendung abgespült werden, wie beispielsweise Shampoos und Rinses, enthalten die beschriebenen Verbindungen gemäss Formel (I) in einer Menge von 1 bis 10 Gew.-%, vorzugsweise 2 bis 6 Gew.-%.

Als übliche Zusatzstoffe in den erfindungsgemässen kosmetischen Zubereitungen kommen beispielsweise kosmetische Harze, Verdicker wie höhere Fettalkohole, Stärke, Cellulosederivate, Paraffinöl, ferner Pflegestoffe, Farbstoffe, Parfümöle, feste Füllstoffe, Emulgatoren, Treibgase und andere in Betracht.

Die nachstehenden Beispiele sollen den Gegenstand der Erfindung näher erläutern.

*Beispiele*

| *Beispiel 1* | Haarwasser |
|---|---|
| 1,0 g | 7-Chlor-3-methyl-2H-[benzo-1,2,4--thiadiazin]-1-dioxid |
| 89,0 g | Isopropanol |
| 9,7 g | Dimethylsulfoxid |
| 0,3 g | Parfümol |
| 100,0 g | |

Das vorstehend angegebene Haarwasser wurde an männlichen und weiblichen Versuchspersonen, die Haarausfall aufwiesen, getestet.

Hierzu wurde jeweils die linke Kopfseite mit dem oben aufgeführten erfindungsgemässen Haarwasser behandelt, während die rechte Kopfseite mit einem sonst identischen, jedoch 7-Chlor-3-methyl--2H-[benzo-1,2,4-thiadiazin]-1-dioxid-freien Haarwasser behandelt wurde. Als Messparameter wurden die subjektive Beobachtung der markierten terminalhaarfreien oder terminalhaararmen Behandlungsstelle, die histometrische Bestimmung des Haarfollikel-Volumens und des Haarwurzelstatus herangezogen.

Es wurde festgestellt, dass bei den männlichen Testpersonen die Behandlung der Kopfhaut mit dem erfindungsgemässen Haarwasser im Verlaufe von 6 bis 10 Monaten den Haarausfall stoppte und darüberhinaus zum subjektiv leicht feststellbaren Wiederwachsen von Terminalhaaren führte. Das Follikelvolumen näherte sich dabei dem für normales Terminalhaar typischen Wert. Der Haarwurzelstatus zeigte, dass nahezu alle Follikel in der aktiven Wachstumsphase standen. Bei den weiblichen Testpersonen wurde eine Verdichtung des Kopfhaares festgestellt. Das Follikelvolumen und der Haarwurzelstatus verhielten sich wie im Falle der männlichen Testpersonen. Demgegenüber konnte an der mit dem 7--Chlor-3-methyl-2H-[benzo-1,2,4-thiadiazin]-1-dioxid-freien Haarwasser behandelten Kopfseite der Testpersonen keine Veränderung des Haarwuchses festgestellt werden.

| *Beispiel 2* | Haarpackung |
|---|---|
| 2,0 g | 7-Chlor-3-methyl-2H-[benzo-1,2,4--thiadiazin]-1-dioxid |
| 30,0 g | Polyoxyethylensorbitanmonopalmitat (Handelsnahme: Tween 40) |
| 57,7 g | Isopropanol |
| 10,0 g | Dimethylsulfoxid |
| 0,3 g | Parfümöl |
| 100,0 g | |

**Patentansprüche**

1. Verwendung eines kosmetischen Mittels auf der Grundlage üblicher kosmetischer Träger- und Zusatzstoffe, gekennzeichnet durch den Gehalt an mindestens einem [Benzo-1,2,4-thiadiazin]-1-dioxid--derivat der allgemeinen Formel

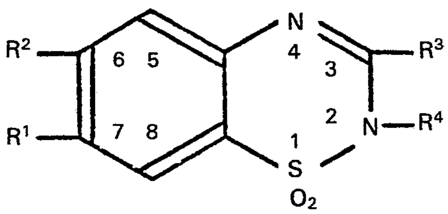

(I)

in der

$R^1$ die Bedeutung Cl, $CF_3$ oder $-SO_2NH_2$ hat,

$R^2$ einer der Reste H, Cl oder $-SO_2NH_2$ ist, wobei jedoch die Reste $R^1$ und $R^2$ nicht beide gleichzeitig Cl oder $-SO_2NH_2$ bedeuten,

$R^3$ einen der Reste H, $C_1$- bis $C_3$-Alkyl, $CH_2Cl$, $CHCl_2$, $CCl_3$, COOH, $-CH_2OH$, $-CH_2OSO_3H$, $-CH_2-CH(CH_3)_2$, $-CH_2-S-CH_2-CF_3$, $-(CH_2)_4-CH_3$, $-CH_2-S-CH_2-CH=CH_2$, $-CH_2-S-CH_2-CH_3$, $-CH(CH_3)-CH(CH_3)-CH_2-CH_3$,

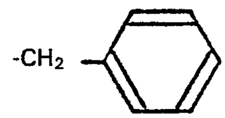 , 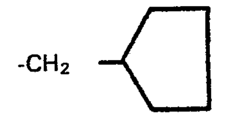

, 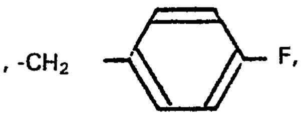

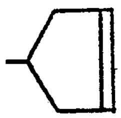 , 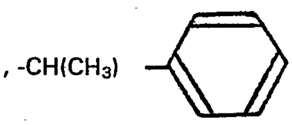 , 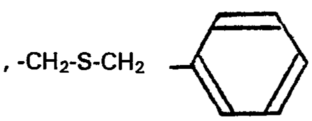

und 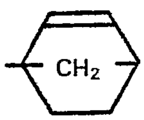 darstellt und

$R^4$ die Bedeutung H oder $CH_3$ hat,
als haarwuchsfördernden Wirkstoff zur Behandlung der Haare und der Kopfhaut.

2. Verwendung eines Mittels nach Anspruch 1, gekennzeichnet durch den Gehalt an mindestens einem [Benzo-1,2,4-thiadiazin]-1-dioxid-derivat der allgemeinen Formel

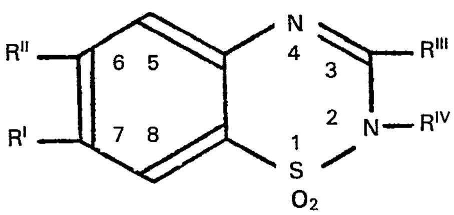

(II)

in der

$R^I$ die Bedeutung Cl, $CF_3$ oder $-SO_2NH_2$ hat,

$R^{II}$ einer der Reste H, Cl oder $-SO_2NH_2$ ist, wobei jedoch die Reste $R^I$ und $R^{II}$ nicht beide gleichzeitig Cl oder $SO_2NH_2$ bedeuten,

$R^{III}$ einen der Reste $CH_3$, $CH_2OH$ und COOH darstellt und

$R^{IV}$ die Bedeutung H oder $CH_3$ hat,
als haarwuchsfördernden Wirkstoff.

3. Verwendung eines Mittels nach Anspruch 1 bis 2, gekennzeichnet durch einen Gehalt an 7-Chlor-3--methyl-2H-[benzo-1,2,4-thiadiazin]-1-dioxid als haarwuchsfördernden Wirkstoff.

4. Verwendung eines Mittels nach Anspruch 1 bis 3, dadurch gekennzeichnet, dass es das [Benzo--1,2,4-thiadiazin]-1-dioxid-derivat gemäss Formel (I) in einer Menge von 0,01 bis 10 Gew.-%, vorzugsweise 0,1 bis 6 Gew.-%, enthält.

5. Verwendung eines Mittels nach Anspruch 1 bis 4, dadurch gekennzeichnet, dass es als Trägerstoff Ethanol, Isopropanol und/oder Dimethylsulfoxid enthält.

6. Verwendung eines Mittels nach Anspruch 1 bis 5, dadurch gekennzeichnet, dass seine Zubereitungsform die einer Lotion, einer Creme oder eines Shampoos ist.

**Claims**

1. Use of a cosmetic medium based on conventional cosmetic carriers and additives, characterised by the content of at least one [benzo-1,2,4-thiadiazine]-1-dioxide derivative of the general formula

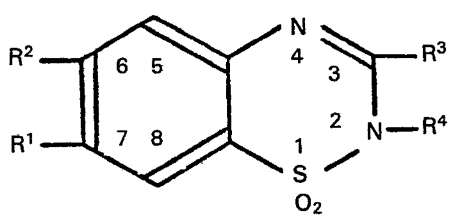

(I)

in which

$R^1$ has the meaning Cl, $CF_3$ or $-SO_2NH_2$,

$R^2$ is one of the residues H, Cl or $-SO_2NH_2$, with the proviso that both of the residues $R^1$ and $R^2$ do not simultaneously mean Cl or $-SO_2NH_2$,

$R^3$ represents one of the residues H, $C_1$- to $C_3$-alkyl, $CH_2Cl$, $CHCl_2$, $CCl_3$, COOH, $-CH_2OH$, $-CH_2OSO_3H$, $-CH_2-CH(CH_3)_2$, $-CH_2-S-CH_2-CF_3$, $-(CH_2)_4-CH_3$, $-CH_2-S-CH_2-CH=CH_2$, $-CH_2-S-CH_2-CH_3$, $-CH(CH_3)-CH(CH_3)-CH_2-CH_3$,

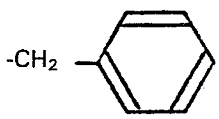 , 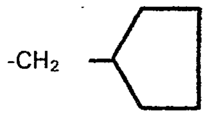 , 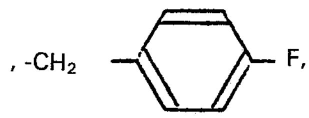

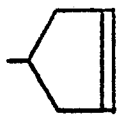 , 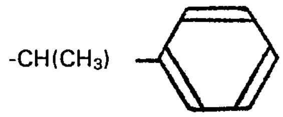 , 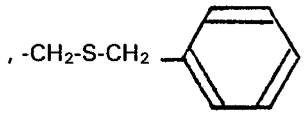

and 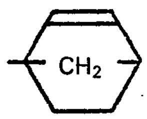

$R^4$ means H or $CH_3$,
as hair-growth promoting agent for the treatment of the hair and scalp.

2. Use of a medium according to claim 1, characterised by the content of at least one [benzo-1,2,4-thiadiazine]-1-dioxide derivative of the general formula

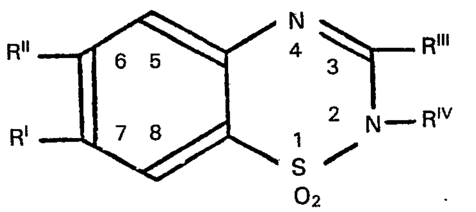

(II)

in which
$R^I$ means Cl, $CF_3$ or $-SO_2NH_2$,
$R^{II}$ is one of the residues H, Cl or $-SO_2NH_2$, with the proviso that both of the residues $R^I$ and $R^{II}$ do not simultaneously mean Cl or $-SO_2NH_2$,
$R^{III}$ represents one of the residues $CH_3$, $CH_2OH$ and COOH,
$R^{IV}$ means H or $CH_3$,
as hair-growth promoting agent.

3. Use of a medium according to claim 1 and 2, characterised by a content of 7-chlor-3-methyl-2H-[benzo-1,2,4-thiadiazine]-1-dioxide as hair-growth promoting agent.

4. Use of a medium according to claim 1 to 3, characterised in that it contains the [benzo-1,2,4-thiadiazine]-1-dioxide derivative according to formula (I) in an amount from 0,01 to 10 weight %, preferably 0,01 to 6 weight %.

5. Use of a medium according to claim 1 to 4, characterised in that it contains, as carrier, ethanol, isopropanol and/or dimethylsulfoxide.

6. Use of a medium according to claim 1 to 5, characterised in that its preparative form is that of a lotion, a creme, or a shampoo.

**Revendications**

1. Utilisation d'un produit cosmétique à base de supports et d'additifs cosmétiques courants, caractérisé en ce qu'il renferme au moins un dérivé de [benzo-1,2,4-thiadiazine]-1-dioxide, répondant à la formule générale

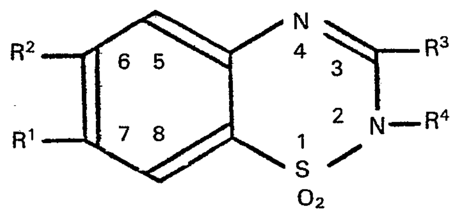

(I)

dans laquelle
$R^1$ représente Cl, $CF_3$ ou $-SO_2NH_2$,
$R^2$ est l'un des restes H, Cl ou $-SO_2NH_2$, les restes $R^1$ et $R^2$ ne représentant cependant pas en même temps Cl ou $-SO_2NH_2$,
$R^3$ représente l'un des restes suivants: H, un alcoyle de C1 à C3, $CH_2Cl$, $CHCl_2$, $CCl_3$, COOH, $-CH_2OH$, $-CH_2OSO_3H$, $-CH_2-CH(CH_3)_2$, $-CH_2-S-CH_2-CF_3$, $-(CH_2)_4-CH_3$, $-CH_2-S-CH_2-CH=CH_2$, $-CH_2-S-CH_2-CH_3$, $-CH(CH_3)-CH(CH_3)-CH_2-CH_3$,

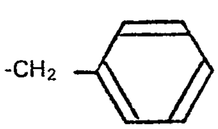 , 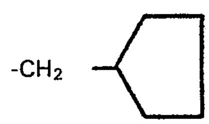 , 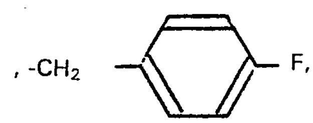

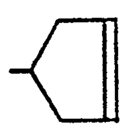 , 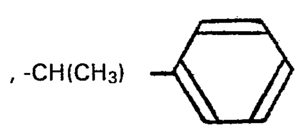 , 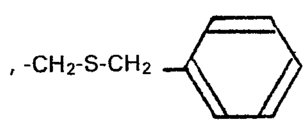

et 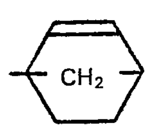 et

$R^4$ représente H ou $CH_3$,
comme principe actif stimulant la croissance des cheveux, pour traiter les cheveux et le cuir chevelu.

2. Utilisation d'un produit selon la revendication 1, caractérisé en ce qu'il renferme au moins un dérivé de [benzo-1,2,4-thiadiazine]-1-dioxyde répondant à la formule générale

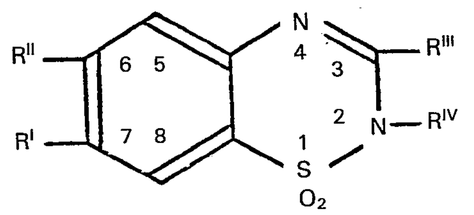

(II)

dans laquelle
$R^I$ Cl, $CF_3$ ou $-SO_2NH_2$,
$R^{II}$ est l'un des restes H, Cl ou $-SO_2NH_2$, les restes $R^I$ et $R^{II}$ ne représentant cependant pas en même temps Cl ou $SO_2NH_2$,
$R^{III}$ représente l'un des restes $CH_3$, $CH_2OH$ et COOH, et
$R^{IV}$ représente H ou $CH_3$,
comme principe actif stimulant la croissance des cheveux.

3. Utilisation d'un produit selon les revendications 1 et 2, caractérisé en ce qu'il renferme du 7-chloro-3-méthyl-2H-[benzo-1,2,4-thiadiazine]-1-dioxyde comme principe actif stimulant la croissance des cheveux.

4. Utilisation d'un produit selon les revendications 1 à 3, caractérisé en ce qu'il renferme le dérivé de [benzo-1,2,4-thiadiazine]-1-dioxyde répondant à la formule I dans une proportion de 0,01 à 10% en poids, de préférence de 0,1 à 6% en poids.

5. Utilisation d'un produit selon les revendications 1 à 4, caractérisé en ce qu'il renferme comme substance porteuse de l'éthanol, de l'isopropanol et/ou du diméthylsulfoxyde.

6. Utilisation d'un produit selon les revendications 1 à 5, caractérisé en ce qu'il est préparé sous la forme d'une lotion, d'une crème ou d'un shampooing.